(19) Europäisches Patentamt
European Patent Office
Office européen des brevets

(11) Publication number: 0 158 374 B1

# (12) EUROPEAN PATENT SPECIFICATION

(45) Date of publication of patent specification: 02.08.89

(51) Int. Cl.⁴: A 01 N 25/04

(21) Application number: 85200266.6

(22) Date of filing: 27.02.85

## (54) Emulsifiable concentrates containing azoles.

(30) Priority: 07.03.84 US 587096

(43) Date of publication of application:
16.10.85 Bulletin 85/42

(45) Publication of the grant of the patent:
02.08.89 Bulletin 89/31

(84) Designated Contracting States:
AT BE CH DE FR GB IT LI LU NL SE

(56) References cited:
EP-A-0 038 109
EP-A-0 057 035
EP-A-0 090 178
EP-A-0 148 526
US-A-3 991 201

(73) Proprietor: JANSSEN PHARMACEUTICA N.V.
Turnhoutsebaan 30
B-2340 Beerse (BE)

(72) Inventor: Ligtvoet, Theo Frans Maria Cornelis
Antwerpseweg 169
B-2151 Vlimmeren (BE)
Inventor: Ruelens, Paul Firmin Marc
Kleine Kruisstraat 4
B-3910-Herk-de-Stad (BE)

EP 0 158 374 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention is concerned with azole-containing formulations which are highly compatible with any aqueous or non-aqueous medium.

1*H*-Imidazoles and 1*H*-1,2,4-triazoles having attractive antimicrobial properties have been described in, for example, U.S. Patent Nos. 3,575,999; 3,717,655; 3,658,813; 3,927,017; 4,156,008; and 4,079,062, and in British Patent Nos. 2,026,486; 2,027,701 and 1,589,852. EP-A-38109 on the other hand describes the use of a group of azole derivatives in the protection and preservation of wood and non-living materials.

Due to their antimicrobal properties the hereinabove-mentioned azoles are very useful in the protection of living subjects and non-living organic materials against decay caused by microorganisms. Living subjects are meant to include human and animal beings as well as plants. Non-living organic materials are meant to include any material which has a substantially organic nature such as, for example, wood, coatings, harvested fruits, foodstuffs, medicines and the like.

The above-mentioned azoles are usually handled in the form of a suitable formulation which may be applied as such or after diluting with a suitable medium. Depending upon the nature of the azole incorporated and the nature of the subjects or materials where the formulation is applied to the said formulations may have a predominantly aqueous or organic nature.

Furthermore, in order to attain sufficient protection it may be desirable to combine an azole with an additional active ingredient such as, for example, a pesticide, e.g., an additional fungicide, insecticide and the like. Said additional active ingredient may be incorporated in the initial formulation or the ultimate mixture may be prepared by adding at least two formulations to an appropriate medium, e.g. an aqueous or an organic medium.

Since each active compound is preferably formulated in a medium appropriate for said compound it is not unusual that by combining two or more active ingredients the resulting formulation does not meet the requirements of stability and the like properties which are necessary or, at least, desirable for handling and/or applying the initial-, respectively the resulting formulation.

The present invention is concerned with azole containing formulations which are highly compatible with any aqueous or non-aqueous medium, said azoles being selected from the group consisting of 1*H*-imidazoles and 1*H*-1,2,4-triazoles having the formula

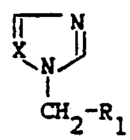

(I)

or an acid addition salt thereof, wherein X is —N= or —CH= and $R_1$ is a radical of the formula

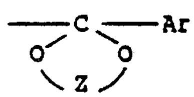

or

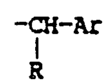

wherein Z is a group $—CH_2—CH_2—$, $—CH_2—CH_2—CH_2—$, $—CH(CH_3)—CH(CH_3)—$ or $—CH_2—CH(alkyl)—$, wherein said alkyl is a straight or branched $C_1—C_{10}$ alkyl radical; said Ar is a phenyl group which is optionally substituted with 1 to 3 halogens, $C_1—C_6$ alkyl radicals, $C_1—C_6$ alkoxy radicals, cyano-, trifluoromethyl- or nitro groups, a thienyl-, halothienyl-, naphthalenyl- or fluorenyl group; and, said R is $C_1—C_{10}$ alkyl, cycloalkyl, cycloalkyllower alkyl, lower alkenyl, aryllower alkyl, aryloxylower alkyl or a radical of the formula $—O—R_o$, wherein said $R_o$ is $C_1—C_{10}$ alkyl, lower alkenyl, lower alkynyl or aryllower alkyl, wherein said aryl radical is phenyl, naphthalenyl or substituted phenyl, wherein said substituted phenyl has 1 to 3 substituents selected from halo, cyano, nitro, phenyl, lower alkyl and lower alkoxy, provided that when more than one substituent is present only one thereof may be cyano, nitro or phenyl.

The subject formulations compatible with any aqueous or non-aqueous medium contain:

i) from 1% to 60% w/v of an azole of formula (I);

ii) from 1% to 60% w/v of a coblock polymer consisting of polyethylene oxide and polypropylene oxide blocks; and

iii) from 1% to 98% w/v of an alkanediol.

Particularly interesting formulations contain an azole of formula

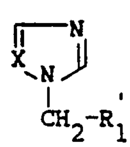

(I-a)

or an acid addition salt thereof, wherein X has the above-identified meaning and $R_1'$ is a radical of the formula

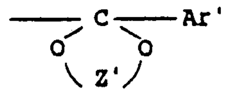 or 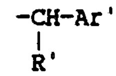

wherein Z′ is a group $—CH_2—CH_2—$, $—CH_2—CH_2—CH_2—$, $—CH(CH_3)—CH_2—$, $—CH(C_2H_5)—CH_2—$, $—CH(C_3H_7)—CH_2—$, $—CH(CH_3)—CH(CH_3)—$ or $CH(CH_3)—CH(C_1H_5)—$; Ar′ is unsubstituted phenyl or phenyl substituted with 1 to 3 halogen atoms, preferably chloro atoms, $C_1—C_6$ alkyl radicals, $C_1—C_6$ alkoxy radicals, cyano or nitro groups; and R′ is $C_1—C_6$ alkyl or $C_3—C_4$ alkenyloxy.

More particularly interesting formulations contain an azole of the formula

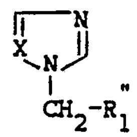 (I-b)

or an acid addition salt thereof, wherein X has the above-identified meaning and $R_1''$ is a radical of the formula

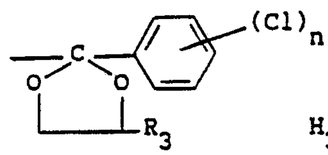 , 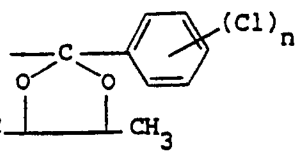 or 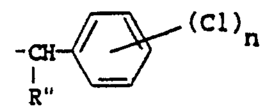

wherein R″ is $C_1—C_4$ alkyl or $C_3—C_4$ lower alkenyloxy, $R_3$ is hydrogen or $C_1—C_3$ alkyl and n is 1 or 2.

Preferred formulations in accordance with the present invention are those containing 1-[2-(2,4-dichlorophenyl)-2-(2-propenyloxy)-ethyl]-1*H*-imidazole, generically designated as imazalil, or a suitable acid addition salt thereof.

The solvent of the formulation is intended to fulfil the requirements of sufficiently solubilizing the azole and, if desired, any additional active ingredient. Furthermore, the solvent must be homogeneously miscible with the emulsifying agent used in the formulation.

Due to their desirable solubilizing effect and their compatibility with a wide range of emulsifying agents alkanedioles such as 1,2-ethanediol, 1,2-propanediol and 1,3-propanediol, are particularly preferred. Even more particularly preferred is 1,2-propanediol since this solvent combines an excellent solubilizing effect with a desirably high flash-point.

Where the selection of the cobloc polymer is less critical as concerns the compatibility of the subject formulations with an organic medium it has been found that the said selection is more critical as concerns the compatibility of the subject formulations with an aqueous medium. Therefore, the more preferred emulsifying agent is a cobloc polymer containing from 30% to 60% of ethylene oxide and having a molar mass of the polypropylene oxide block of 950 to 5000 g per mole.

The most preferred cobloc polymer contains 50% of polyethylene oxide and has a molar mass of 3250 polypropylene oxide block per mole.

In addition to the azole, a possible additional active ingredient, a solvent and an emulsifying agent for formulations may also contain one or more additives. Such additives may be added to the formulations in order to ameliorate the properties of the initial formulations or of the formulations finally applied.

A suitable additive is, for example, a co-emulsifying agent which has the effect that by mixing the initial formulation with an aqueous or organic medium the resulting dilution is much more stable. Suitable co-emulsifying agents are, for example, $(C_6—C_{34})$alkyl benzenesulfonic acid.

In the formulations of the present invention the azoles can also be used in combination with other compounds having useful activity as, biocidal compounds, e.g. antimicrobal agents, insecticides and the like.

As antimicrobial agents, which may be used in combination with the azoles there may be considered products of the following classes:

Phenol derivatives such as 3,5-dichlorophenol, 2,5-dichlorophenol, 3,5-dibromophenol, 2,5-dibromophenol, 2,5-(resp. 3,5)-dichloro-4-bromophenol, 3,4,5-trichlorophenol; chlorinated hydrodiphenylethers such as, for example, 2-hydroxy-3,2′4′-trichlorodiphenylether, phenylphenol, 4-chloro-2-phenylphenol, 4-chloro-2-benzylphenol, dichlorophene, hexachlorophene; aldehydes such as formaldehyde, glutaraldehyde, salicylaldehyde; alcohols such as phenoxyethanol; antimicrobially active carboxylic acids and their derivatives; organometallic compounds such as tributyltin compounds; iodine compounds such as iodophores, iodonium compounds; mono-, di and polyamines such as dodecylamine or 1,10-di(n-heptyl)-1,10-diaminodecane; quaternary ammonium compounds such as benzyl-dimethyldode-cylammonium chloride, dimethyldodecylammonium chloride, benzyldi(2-hydroxyethyl)-dodecylammonium choride; sulfonium and phosphonium compounds; mercapto compounds as well their alkali, earth alkaline and heavy metal salts such as 2-mercaptopyridine-*N*-oxide and its sodium and zinc

salt, 3-mercaptopyridazin-2-oxide, 2-mercaptoquinoxaline-1-oxide, 2-mercaptoquinoxaline-di-*N*-oxide, as well as the symmetrical disulfides of said mercapto compounds; ureas such as tribromo or trichlorocarbanilide, dichlorotrifluoromethyldiphenylurea; tribromosalicylanilide; 2-bromo-2-nitro-1,3-dihydroxypropane; dichlorobenzoxazolon; chlorohexidine; isothia- and benzisothiazolone derivatives.

As insecticidal agents which may be used in combination with the azoles the following classes of products may be considered: insecticides having a natural origin, e.g., nicotine, rotenone, pyrethrum and the like; chloridinated hydrocarbons, e.g., lindane, chlordane, endosulfan and the like; organic phosphor compounds; carbamates, e.g., carbaryl, aldicarb, methiocarb, propoxur and the like; biological insecticides, e.g., products originating from *Bacillus thuringiensis;* synthetic pyrethroids, e.g., permethrin, allethrin, cypermethrin, halothrin and the like.

Besides their high compatibility with aqueous and non-aqueous mediums the subject formulations are particularly attractive for incorporating relatively high doses of azoles and, if desired, other active ingredients.

Preferred concentrated formulations contain from 20% to 60% w/v of an azole.

By combining a high compatibility for aqueous and non-aqueous mediums with a capacity for incorporating relatively high doses of azoles and, if desired, other active ingredients the subject formulations are particularly attractive for handling concentrated azole containing mixtures which are usually applied to subjects or materials in relatively diluted mixtures. Diluted mixtures are meant to include for example tank-mixes for administering an azole and, if desired, an additional active ingredient to subjects or materials. Depending upon the application technique and the nature of the subject or material where the azole, optionally combined with an additional active ingredient, is applied to the diluted mixture may be any liquid, such as, for example, aqueous- or non-aqueous mixtures, e.g. aqueous or non-aqueous dilutions, aqueous- or non-aqueous waxes and the like liquids.

The subject formulations have also the advantage that the activity of the azoles incorporated therein is positively influenced. As a consequence thereof analogous antimicrobial activities may be obtained with reduced concentrations of the azole in the mixture applied to the subject or material.

Additionally, compared with the art-known formulations the subject formulations effect also a reduced phytotoxicity of the mixtures ultimately applied to, resulting in a very useful application mixture with a desirable safety margin.

The hereinabove mentioned properties, combined with a substantial lack of irritation render the subject formulations particularly attractive, not only for their desirable safety margin for the subjects or materials applied to but also for the applier.

The subject formulations may be prepared by mixing the components together at a temperature between 10°C and 100°C, preferably between 15°C and 60°C.

The following examples are intended to illustrate and not to limit the scope of the present invention. Unless otherwise stated herein all parts therein are by weight.

Pluriol PE®, as used in the experimental part, is a Trade Mark for a cobloc polymer consisting of polyethylene oxide- and polypropylene oxide blocks wherein the molar mass of the polypropylene oxide block is 3250 g per mole and 50% of the cobloc-polymer consists of polyethylene oxide blocks.

## EXAMPLES

### Example 1: Preparation of emulsifiable concentrates

formulation 1: 50% w/v imazalil;
14.5% w/v Pluriol PE®;
7.15% w/v dodecylbenzenesulfonic acid; and
1,2-propanediol ad volume.

Preparation:

50 Parts of imazalil were mixed with 20 parts of 1,2-propanediol at 50°C. Subsequently 14.5 parts of Pluriol PE® and 7.15 parts of dodecylbenzenesulfonic acid were added and the mixture was stirred at 20°C until the mixture was homogeneous. Finally, the mixture was diluted to 100 ml.

formulation 2: 10% w/v imazalil;
1% w/v cypermethrin;
5% w/v Pluriol PE®; and
1,2-propanediol ad 100 ml.

Preparation:

10 Parts of imizalil were mixed with 50 parts of 1,2-propandiol. Subsequently 1 part of cypermethrin and 5 parts of Pluriol PE® were added and the mixture was stirred at 20°C until the mixture was homogeneous. Finally, the mixture was diluted to 100 ml.

### Example 2: Antifungal activity

Young cucumber plants, about 10 days old, were sprayed with a mixture containing 10, 1 or 0.1 ppm of imazalil while controls were kept untreated. After drying of the plants artificial infection with spores of *Erysiphe cichoracearum* was carried out by slightly rubbing the plants with a heavily infected leaf. At the

15th day after artificial infection the degree of fungal attack was evaluated by counting the number of spots per plant.

The results given in Table 1 are the percentages of fungal attack in comparison with the untreated plants. Dilution A was prepared by diluting a xylene-based emulsifiable concentrate containing 20% of imazalil with water. Dilution B was prepared by diluting formulation 1 (described hereinafter) with water.

Table 1: percentage of fungal attack

| | concentration in ppm | | |
|---|---|---|---|
| | 10 | 1 | 0.1 |
| dilution A | 0 | 20 | 50 |
| dilution B | 0 | 0 | 1 |

Example 3: Phytotoxicity-tests

Pears (Doyenné de Comice and Conference) were immersed into an imazalil containing mixture during two minutes. After pears were leaked out during one minute they were placed on a glass petridish and stored during 5 days at 5°C. As the apples and pears are not dried before storage a moistened ring is formed between the glass petridish and the contact-surface of the pear. Phytotoxicity was measured by determining the percent of the contact-surface which shows any form of decay or discoloration.

The results given in Table 2 are the percentages of the contact-surface which shows any form of decay or discoloration at different concentrations of imazalil in the immersion mixture.

Apples were immersed in an imazalil containing mixture for two minutes. After the apples had stopped dripping after one minute they were placed five in a glass recipient of five litre and stored for 5 days at 2°C. As the apples were not dried before storage a moistened ring was formed at the contact-surfaces of the apples and the contact-surfaces formed between the apples and the glass recipient. Phytotoxicity was measured by determining the percent of the contact-surface which shows any form of decay or discoloration.

The results given in Table 3 are the percentages of the contact-surface which shows any form of decay or discoloration at different concentrations of imazalil in the immerse-mixture.

The imazalil-containing mixtures under investigation were:

— dilution C, prepared by diluting formulation 1 (described hereinabove) with water;
— dilution D, prepared by diluting a water-soluble powder containing 75% of imazalil sulfate with water; and
— dilution E, prepared by diluting a xylene-based emulsifiable concentrate containing 20% of imazalil with water.

TABLE 2

Phytotoxicity data on pears

| | Percent of decay or discoloration of the contact-surface | |
|---|---|---|
| | Doyenné du Comice | Conference |
| dilution C | | |
| 1000 ppm | 2 | 0 |
| 1500 ppm | 2 | 0 |
| 2000 ppm | 2 | 0 |
| dilution E | | |
| 250 ppm | 2 | 0 |
| 500 ppm | 5 | 1 |
| 1000 ppm | 6 | 2 |

TABLE 3

Phytotoxicity data on apples

| | Percent of decay or discoloration of the contact-surface |
|---|---|
| | Golden delicious |
| dilution C | |
| 1000 ppm | 0 |
| 2000 ppm | 0.5 |
| dilution D | |
| 1000 ppm | 2 |

Example 4: Physical compatibility

Dilutions containing 500 ppm of imazalil and 500 ppm of a mix-pesticide are prepared by diluting the initial mix-pesticide containing formulations with standard hard water (342 ppm) at 30°C, adding a calculated amount of formulations 1 and, finally, diluting with standard hard water up to the desired concentration of imazalil and mix-pesticide containing formulations with standard hard water (342 ppm) at 30°C. Blanco formulations containing only the mix-pesticide were also prepared following the same procedure. The samples were inverted until a homogeneous mixture was obtained (number of inversion-cycles: see Table 4, column 1 for blanco and column 2 for the combination) and, subsequently, up to twenty inversion-cycles. After an immobile storage of the samples at 30°C the samples were inverted until any sedimentation or creaming had completely disappeared (number of inversion-cycles: see Table 4, column 3 for blanco and column 4 for the combination).

Table 4

| | number of inversion-cycles | | | |
|---|---|---|---|---|
| mix-pesticide containing formulation | column 1 blanco | column 2 combination | column 3 blanco | column 4 combination |
| Vinclozolin 50% WP* | 2 | 2 | 1 | 1 |
| Thiophanate 70% WP | 1 | 2 | 1 | 2 |
| 3-(3,5-dichlorophenyl)-1-isopropylcarbamoxyl-hydantoin 50% WP | 1 | 2 | 1 | 2 |
| Carbendazin 50% WP | 2 | 2 | 2 | 2 |
| Triadimefon 25% WP | 3 | 3 | 2 | 3 |
| Carbendazin 50% FL* | 3 | 3 | 6 | 7 |
| Thiabendazol 45% FL | 3 | 8 | 2 | x |

* : WP is an abbreviation for wettable powder

* : FL is an abbreviation for flowable

**Claims**

1. An emulsifiable concentrate containing

i) from 1% to 60% w/v of a coblock polymer consisting of polyethylene oxide and polypropylene oxide blocks;

ii) from 1% to 98% w/v of an alkanediol; and

iii) from 1% to 60% w/v of one azole having the formula

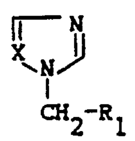

(I)

or an acid addition salt thereof, wherein X is —N= or —CH= and $R_1$ is a radical of the formula

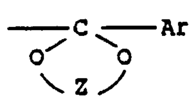

or

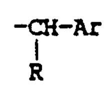

wherein Z is a group $-CH_2-CH_2-$, $-CH_2-CH_2-CH_2-$, $-CH(CH_3)-CH(CH_3)-$ or $-CH_2-CH(alkyl)-$, wherein said alkyl is a straight or branced $C_1-C_{10}$ alkyl radical; said Ar is a phenyl group which is optionally substituted with 1 to 3 halogens, $C_1-C_6$ alkyl radicals, $C_1-C_6$ alkoxy radicals, cyano-, trifluoromethyl- or nitro groups, a thienyl-, halothienyl-, naphthalenyl- or fluorenyl group; and, said R is $C_1-C_{10}$ alkyl, cycloalkyl, cycloalkyllower alkyl, lower alkenyl, aryllower alkyl, aryloxylower alkyl or a radical of the formula $-O-R_o$, wherein said $R_o$ is $C_1-C_{10}$ alkyl, lower alkenyl, lower alkynyl or aryllower alkyl, wherein said aryl radical is phenyl, naphthalenyl or substituted phenyl, wherein said substituted phenyl has 1 to 3 substituents selected from halo, cyano, nitro, phenyl, lower alkyl and lower alkoxy, provided that when more than one substituent is present only one thereof may be cyano, nitro or phenyl.

2. An emulsifiable concentrate according to claim 1 wherein the azole is selected from the compounds having the formula

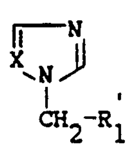

(I-a)

or an acid addition salt thereof, wherein X is —N= or —CH= and $R_1'$ is a radical of the formula

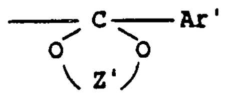

or

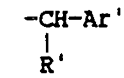

wherein Z' is a group $—CH_2—CH_2—$, $—CH_2—CH_2—CH_2—$, $—CH(CH_3)—CH_2—$, $—CH(C_2H_5)—CH_2—$, $—CH(C_3H_7)—CH_2—$, $—CH(CH_3)—CH(CH_3)—$ or $—CH(CH_3)—CH(C_1H_5)—$; Ar' is unsubstituted phenyl or phenyl substituted with 1 to 3 halogen atmos, preferably chloro atoms, $C_1—C_6$ alkyl radicals, $C_1—C_6$ alkoxy radicals, cyano or nitro groups; and R' is $C_1—C_6$ alkyl or $C_3—C_4$ alkenyloxy.

3. An emulsifiable concentrate according to claim 1 wherein the azole is selected from the compounds having the formula

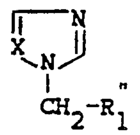

(I-b)

or an acid addition salt thereof, wherein X is —N= or —CH= and $R_1''$ is a radical of the formula

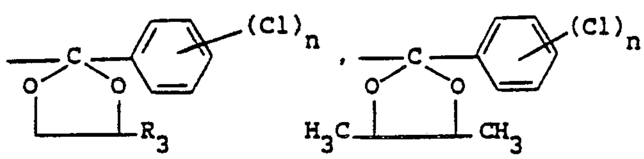

or

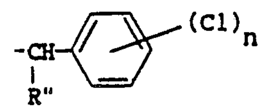

wherein R'' is $C_1—C_4$ alkyl, $C_3—C_4$ lower alkenyloxy, $R_3$ is hydrogen or $C_1—C_3$ alkyl and n is 1 or 2.

4. An emulsifiable concentrate according to claim 1 wherein the azole is 1-[2-(2,4-dichlorophenyl)-2-(2-propenyloxy)ethyl]-1*H*-imidazole or an acid addition salt thereof.

5. An emulsifiable concentrate according to any of claims 1 to 4 wherein the solvent is 1,2-ethanediol or 1,2-propanediol.

6. An emulsifiable concentrate according to claim 5 wherein the solvent is 1,2-propanediol.

7. An emulsifiable concentrate according to any of claims 1 to 6 containing additionally from 0.5% to 15% of a co-emulsifying agent.

8. An emulsifiable concentrate according to claim 7 wherein the co-emulsifying agent is selected from the group consisting of $(C_6—C_{34})$ alkyl benzenesulfonic acids.

9. An emulsifiable concentrate containing

i) from 10% to 20% w/v of a cobloc polymer consisting of polyethylene oxide and polypropylene oxide blocks;

ii) from 45% to 55% w/v of (±)-1-[2-(2,4-dichlorophenyl)-2-(2-propenyloxy)ethyl]-1*H*-imidazole; and

iii) 1,2-propanediol ad volume.

10. An emulsifiable concentrate according to claim 12 containing from 5% to 10% w/v of a co-emulsifying agent selected from the group consisting of $(C_6—C_{34})$alkyl benzenesulfonic acids.

11. An emulsifiable concentrate containing

i) 50% w/v of (±)-1-[2-(2,4-dichlorphenyl)-2-(2-propenyloxy)ethyl]-1*H*-imidazole;

ii) 14.5% w/v of a cobloc polymer consisting of polyethylene oxide- and polypropylene oxide blocks wherein the molar mass of the polypropylene oxide block is 3250 g per mole and 50% of the cobloc polymer consists of polyethylene oxide blocks;

iii) 7.15% w/v of dodecylbenzenesulfonic acid; and

iv) 1,2-propanediol ad volume.

12. A mixture prepared by diluting an emulsifiable concentrate according to any of claims 1 to 10; for use in the protection of harvested fruits, vegetables and foodstuffs.

13. A process for preparing an emulsifiable concentrate according to claim 1, characterized by solubilizing the azole in part of the solvent at a temperature between 10°C and 100°C, subsequently mixing the thus obtained mixture with the emulsifying agent at a temperature between 10°C and 100°C, if desired, adding a suitable additive and, finally, diluting the mixture with solvent ad volume at a temperature between 10°C and 100°C.

**Patentansprüche**

1. Emulgierbares Konzentrat, enthaltend

i) von 1% bis 60% Gewicht/Volumen eines Coblockpolymers, bestehend aus Polyethylenoxid- und Polypropylenoxidblöcken;

ii) von 1% bis 98% Gewicht/Volumen eines Alkandiols; und

iii) von 1% bis 60% Gewicht/Volumen eines Azols der Formel

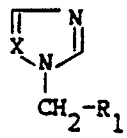

(I)

oder eines Säureadditionssalzes hievon, worin X für —N= oder —CH= steht und $R_1$ ein Rest der Formel

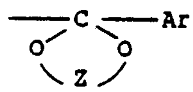 or 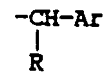

ist, worin Z eine $—CH_2—CH_2—$, $—CH_2—CH_2—CH_2—$, $—CH(CH_3)—CH(CH_3)—$ oder $—CH_2—CH(Alkyl)$-Gruppe darstellt, worin das genannte Alkyl ein linearer oder verzweigter $C_1—C_{10}$Alkylrest ist; das genannte Ar eine Phenylgruppe darstellt, welche wahlweise mit 1 bis 3 Halogenatom, $C_1—C_6$Alkylresten, $C_1—C_6$Alkoxyresten, Cyano, Trifluormethyl- oder Nitrogruppen einer Thienyl-, Halothienyl-, Naphthalenyl- oder Fluorenylgruppe substituiert ist; und das genannte R für $C_1—C_{10}$Alkyl, Cycloalkyl, Cycloalkylniederalkyl, Niederalkenyl, Arylniederalkyl, Aryloxyniederalkyl oder einen Rest der Formel $—O—R_0$ steht, worin das genannte $R_0$ für $C_1—C_{10}$Alkyl, Niederalkenyl, Niederalkinyl oder Arylniederalkyl steht, worin der genannte Arylrest Phenyl, Naphthalenyl oder ein substituiertes Phenyl ist, worin das genannte substituierte Phenyl 1 bis 3 unabhängig voneinander aus Halogen, Cyano, Nitro, Phenyl, Niederalkyl und Niederalkoxy ausgewählte Substituenten besitzt, mit der Maßgabe, daß bei Vorliegen von mehr als einem Substituenten nur einer hievon Cyano, Nitro oder Phenyl sein kann.

2. Emulgierbares Konzentrat nach Anspruch 1, worin das Azol unter den Verbindungen mit der Formel

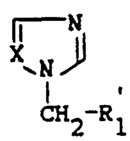

(I-a)

oder einem Säureadditionssalz hievon ausgewählt ist, worin X für —N= oder —CH= steht und $R_1'$ ein Rest der Formel

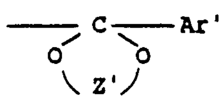 or 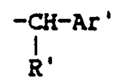

ist, worin Z' eine $—CH_2—CH_2—$, $—CH_2—CH_2—CH_2—$, $—CH(CH_3)—CH_2—$, $—CH(CH_2H_5)—CH_2—$, $—CH(C_3H_8)—CH_2—$, $—CH(CH_3)—CH(CH_3)—$ oder $—CH(CH_3)—CH(C_2H_5)$-Gruppe darstellt; Ar' ein unsubstituiertes Phenyl oder ein mit 1 bis 3 Halogenatomen, vorzugsweise Chloratomen, $C_1—C_6$Alkylresten, $C_1—C_6$Alkoxyresten, Cyano- oder Nitrogruppen substituiertes Phenyl ist; und R' für $C_1—C_6$Alkyl oder $C_3—C_4$Alkenyloxy steht.

3. Emulgierbares Konzentrat nach Anspruch 1, worin das Azol unter Verbindungen der Formel

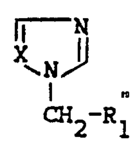

(I-b)

oder einem Säureadditionssalz hievon ausgewählt ist, worin X für —N= oder —CH= steht und $R_1''$ ein Rest der Formel

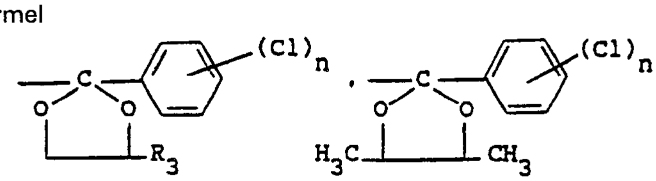 oder 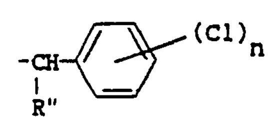

ist, worin R'' für $C_1—C_4$Alkyl, $C_3—C_4$Niederalkenyloxy steht, $R_3$ Wasserstoff oder $C_1—C_3$ Alkyl ist und n den Wert 1 oder 2 aufweist.

4. Emulgierbares Konzentrat nach Anspruch 1, worin das Azol 1-[2-(2,4-Dichlorphenyl)-2-(2-propenyloxy)ethyl]-1*H*-imidazol oder ein Säureadditionssalz hievon ist.

5. Emulgierbares Konzentrat nach einem der Ansprüche 1 bis 4, worin das Lösungsmittel 1,2-Ethandiol oder 1,2-Propandiol ist.

6. Emulgierbares Konzentrat nach Anspruch 5, worin das Lösungsmittel 1,2-Propandiol ist.

7. Emulgierbares Konzentrat nach einem der Ansprüche 1 bis 6, welches zusätzlich von 0,5% bis 15% eines co-emulgierenden Mittels enthält.

8. Emulgierbares Konzentrat nach Anspruch 7, worin das co-emulgierende Mittel aus der aus ($C_6$—$C_{34}$)Alkylbenzolsulfonsäuren bestehenden Gruppe ausgewählt ist.

9. Emulgierbares Konzentrat, welches

i) von 10% bis 20% Gewicht/Volumen eines Coblockpolymers, bestehend aus Polyethylenoxid- und Polypropylenoxidblöcken;

ii) von 45% bis 55% Gewicht/Volumen (±)-1-[2-(2,4-Dichlorphenyl)-2-(2-propenyloxy)ethyl]-1*H*-imidazol; und

iii) 1,2-Propandiol ad Vol.

enthält.

10. Emulgierbares Konzentrat nach Anspruch 9, welches von 5% bis 10% Gewicht/Volumen eines aus der aus ($C_6$—$C_{34}$)Alkylbenzolsulfonsäuren bestehenden Gruppe ausgewählten co-emulgierenden Mittels enthält.

11. Emulgierbares Konzentrat, welches

i) 50% Gewicht/Volumen (±)-1-[2-(2,4-Dichlorphenyl)-2-(2-propenyloxy)ethyl]-1*H*-imidazol;

ii) 14,5% Gewicht/Volumen eines Coblockpolymers, bestehend aus Polyethylenoxid- und Polypropylenoxidblöcken, worin die Molmasse des Polypropylenblockes 3.250 g pro Mol beträgt und 50% des Coblockpolymers aus Polyethylenoxidblöcken bestehen;

iii) 7,15% Gewicht/Volumen Dodecylbenzolsulfonsäure; und

iv) 1,2-Propandiol ad Vol.

enthält.

12. Durch Verdünnen eines emulgierbaren Konzentrats nach einem der Ansprüche 1 bis 11 hergestelltes Gemisch zur Anwendung im Schutz von geernteten Früchten, Gemüssen und von Nahrungsmitteln.

13. Verfahren zur Herstellung eines emulgierbaren Konzentrates nach Anspruch 1, gekennzeichnet durch Auflösen des Azols in einem Teil des Lösungsmittels bei einer Temperatur von 10°C bis 100°C, darauf folgendes Vermischen des so erhaltenen Gemisches mit dem emulgierenden Mittel bei einer Temperatur von 10°C bis 100°C, wenn gewünscht, Zugabe eines entsprechenden Additivs und abschließend Verdünnen des Gemisches mit Lösungsmittel ad Vol. bei einer Temperatur von 10°C bis 100°C.

**Revendications**

1. Concentré émulsionnable contenant

i) de 1% à 60% en poids/volume d'un polymère co-séquencé constitué par des blocs de poly(oxyde d'éthylène) et de poly(oxyde de propylène);

ii) de 1% à 98% en poids/volume d'un alcanediol; et

iii) de 1% à 60% en poids/volume d'un azole de formule

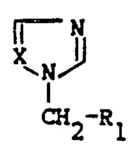

(I)

ou un de leurs sels d'addition avec un acide, formule dans laquelle X est —N= ou —CH= et $R_1$ est un radical de formule

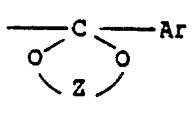

ou

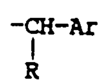

formules dans lesquelles Z est $—CH_2—CH_2—$, $—CH_2—CH_2—CH_2—$, $—CH(CH_3)—CH(CH_3)—$ ou $—CH_2—$CH(alkyle)-, dans lequel ledit alkyle est un radical alkyle en $C_1$—$C_{10}$ linéaire ou ramifié; ledit Ar est un groupe phényle qui est éventuellement substitué par 1 à 3 atomes d'halogène, des radicaux alkyle en $C_1$—$C_6$, des radicaux alcoxy en $C_1$—$C_6$, des groupes cyano, trifluorométhyle ou nitro, un groupe thiényle, halogénothiényle, naphtalényle ou fluorényle; et ledit R est un groupe alkyle en $C_1$—$C_{10}$, cycloalkyle, cycloalkyl(alkyle inférieur), alcényle inférieur, aryl(alkyle inférieur), aryloxy(alkyle inférieur), ou un radical de formule —O—$R_o$, dans laquelle $R_o$ est un groupe alkyle en $C_1$—$C_{10}$, alcényle inférieur, alcynyle inférieur ou aryl(alkyle inférieur), où ledit radical aryle est un groupe phényle, naphtalényle ou phényle substitué, dans lequel ledit groupe phényle substitué comporte 1 à 3 substituants choisis indépendamment parmi les groupes halogéno, cyano, nitro, phényle, alkyle inférieur et alcoxy inférieur, à condition que, lorsque plus d'un substituant est présent, l'un deux seulement puisse être un groupe cyano, nitro ou phényle.

2. Concentré émulsionnable selon la revendication 1, dans lequel l'azole est choisi parmi les composés de formule

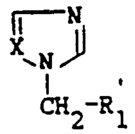

(I-a)

ou un de leurs sels d'addition avec un acide, formule dans laquelle X est —N= ou —CH= et $R_1'$ est un radical de formule

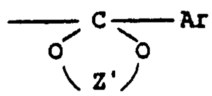
or
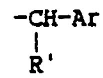

formules dans lesquelles Z' est un groupe $—CH_2—CH_2—$, $—CH_2—CH_2—CH_2—$, $—CH(CH_3)—CH_2—$, $—CH(C_2H_5)—CH_2—$, $—CH(C_3H_7)—CH_2—$, $—CH(CH_3)—CH(CH_3)—$ ou $—CH(CH_3)—CH(C_2H_5)—$; Ar' est un groupe phényle non substitué ou phényle substitué par 1 à 3 atomes d'halogène, de préférence des atomes de chlore, des radicaux alkyle en $C_1—C_6$, des radicaux alcoxy en $C_1—C_6$, des groupes cyano ou nitro; et R' est un groupe alkyle en $C_1—C_6$ ou alcényloxy en $C_3—C_4$.

3. Concentré émulsionnable selon la revendication 1, dans lequel l'azole est choisi parmi les composés de formule

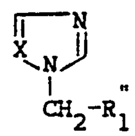

(I-b)

ou un de leurs sels d'addition avec un acide, formule dans laquelle X est —N= ou —CH= et $R_1''$ est un radical de formule

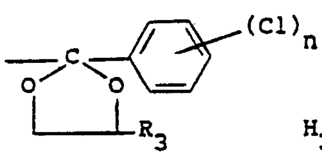
,
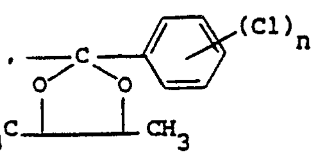
or
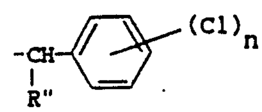
.

formules dans lesquelles R'' est un groupe alkyle en $C_1—C_4$ ou alcényloxy inférieur en $C_3—C_4$, $R_3$ est un hydrogène ou un groupe alkyle en $C_1—C_3$ et n est égal à 1 ou 2.

4. Concentré émulsionnable selon la revendication 1, dans lequel l'azole est le 1-[2-(2,4-dichlorphényl)-2-(2-propényloxy)éthyl]-1H-imidazole ou un de ses sels d'addition avec un acide.

5. Concentré émulsionnable selon l'une quelconque des revendications 1 à 4, dans lequel le solvant est le 1,2-éthanediol ou le 1,2-propanediol.

6. Concentré émulsionnable selon la revendication 5, dans lequel le solvant est le 1,2-propanediol.

7. Concentré émulsionnable selon l'une quelconque des revendications 1 à 6, contenant en plus de 0,5% à 15% d'un agent co-émulsionnant.

8. Concentré émulsionnable selon la revendication 7, dans lequel l'agent co-émulsionnant est choisi dans le groupe constitué par les acides alkyl(en $C_6—C_{34}$)benzènesulfoniques.

9. Concentré émulsionnable contenant

i) de 10% à 20% en poids/volume d'un polymère co-séquencé constitué par des blocs de poly(oxyde d'éthylène) et de poly(oxyde de propylène);

ii) de 45% à 55% en poids/volume de (±)-1-[2-(2,4-dichlorophényl)-2-(2-propényloxy)éthyl]-1*H*-imidazole; et

iii) du 1,2-propanediol en quantité suffisante pour compléter au volume.

10. Concentré émulsionnable selon la revendication 9, contenant de 5% à 10% en poids/volume d'un agent co-émulsionnant choisi dans le groupe constitué par les acides alkyl(en $C_6—C_{34}$)benzènesulfoniques.

11. Concentré émulsionnable contenant

i) 50% en poids/volume de (±)-1-[2-(2,4-dichlorophényl)-2-(2-propényloxy)éthyl]-1*H*-imidazole;

ii) 14,5% en poids/volume d'un polymère co-séquencé constitué par des blocs de poly(oxyde d'éthylène) et de poly(oxyde de propylène), dans lequel la masse molaire du bloc de poly(oxyde de propylène) est de 3250 g par mole et 50% du polymère co-séquencé est constitué par des blocs de poly(oxyde d'éthylène);

iii) 7,15% en poids/volume d'acide dodécylbenzènesulfonique, et

iv) du 1,2-propanediol en quantité suffisante pour compléter au volume.

12. Mélange préparé par dilution d'un concentré émulsionnable selon l'une quelconque des

revendications 1 à 11, utilisable dans la protection des fruits récoltés, des légumes et des denrées alimentaires.

13. Procédé de préparation d'un concentré émulsionnable selon la revendication 1, caractérisé en ce que l'on solubilise l'azole dans une partie du solvant à une température comprise entre 10°C et 100°C, on mélange ensuite le mélange ainsi obtenu avec l'agent émulsionnant à une température comprise entre 10°C et 100°C, on ajoute éventuellement un additif convenable et, enfin, on dilue le mélange avec le solvant pour compléter au volume à une température comprise entre 10°C et 100°C.